# EUROPEAN PATENT SPECIFICATION

(11) **EP 1 660 662 B1**
(45) Date of publication and mention of the grant of the patent: **21.04.2010**
(21) Application number: 04766676.3
(22) Date of filing: 02.09.2004
(51) Int. Cl.: C12N 15/85

(54) **REGULATABLE GENE EXPRESSION IN MAMMALIAN CELLS AND MAMMALS**
REGULIERBARE GENEXPRESSION IN SÄUGERZELLEN UND SÄUGERN
EXPRESSION CONTROLABLE DES GENES DANS DES CELLULES DE MAMMIFERES ET DES MAMMIFERES

(30) Priority: 02.09.2003 EP 03405635
(43) Date of publication of application: 31.05.2006
(73) Proprietor: Fussenegger, Martin, 8046 Zürich (CH); Weber, Wilfried, 8049 Zürich (CH)
(72) Inventor: Fussenegger, Martin, 8046 Zürich (CH); Weber, Wilfried, 8049 Zürich (CH)
(74) Representative: Becker, Konrad
(86) International application number: PCT/EP2004/051997
(87) International publication number: WO 2005/021766

(56) References cited:
- WO-A-01/09357
- WO-A-01/31047
- WO-A-97/06269
- CHEN ANPING: "Acetaldehyde stimulates the activation of latent transforming growth factor-beta1 and induces expression of the type II receptor of the cytokine in rat cultured hepatic stellate cells." THE BIOCHEMICAL JOURNAL. ENGLAND 15 DEC 2002, vol. 368, no. Pt 3, 15 December 2002 (2002-12-15), pages 683-693, XP002267295 ISSN: 0264-6021 cited in the application
- MRH WHITE: INTERNET ARTICLE, [Online] 11 November 1999 (1999-11-11), page 5, XP002265633 Retrieved from the Internet: URL:http://www.liv.ac.uk/~bates/MolBiol/Pr ojects98.html> [retrieved on 2003-12-17]
- FLIPPHI MICHEL ET AL: "Characteristics of physiological inducers of the ethanol utilization (alc) pathway in Aspergillus nidulans" BIOCHEMICAL JOURNAL, vol. 364, no. 1, 15 May 2002 (2002-05-15), pages 25-31, XP002265634 ISSN: 0264-6021 cited in the application
- SMITS THEO H M ET AL: "New alkane-responsive expression vectors for Escherichia coli and Pseudomonas" PLASMID, vol. 46, no. 1, July 2001 (2001-07), pages 16-24, XP002265635 ISSN: 0147-619X cited in the application
- WEBER WILFRIED ET AL: "Gas-inducible transgene expression in mammalian cells and mice." NATURE BIOTECHNOLOGY. NOV 2004, vol. 22, no. 11, November 2004 (2004-11), pages 1440-1444, XP002310266 ISSN: 1087-0156
- CADDICK M X: "An ethanol inducible gene switch for plants used to manipulate carbon metabolism" NATURE BIOTECHNOLOGY, vol. 16, February 1998 (1998-02), pages 177-180, XP000764088
- SALTER M G: "Characterization of the ethanol-inducible alc gene expression system for transgenic plants" THE PLANT JOURNAL, vol. 16, no. 1, 1998, pages 127-132,

## Description

### Technical Field

The invention relates to mammalian cells and non-human mammals harboring a protein which transactivates transcription from natural or chimeric promoters in response to compounds being gaseous or liquid at cultivation temperature or at the body temperature of the mammal. Furthermore, the invention relates to a method of using such proteins to regulate gene expression in mammalian cell culture or non-human mammals in response to regulating compounds being gaseous or liquid at cultivation temperature or at the body temperature of the mammal. The invention further discloses nucleic acids useful for constructing said mammalian cells and mammals.

### Background Art

Current technologies for regulating gene expression in mammalian cells or mammals require administration of small-molecule drugs, typical examples of which are antibiotics, hormone analogues or immunosupressive agents (see e.g. US Pat. No. 6,287,813, US Pat. No. 6,379,945, US Pat. No. 6,187,757, US Pat. No. 5,464,758). These compounds are often difficult to remove from the cell and the cell's environment after administration, which impedes reversion of the gene expression status. Also, regulating small-molecule drugs elicit long-term side effects on human or animal organisms, which raises safety concerns in gene therapy trials.

Both limitations can be overcome by novel gene regulation technologies, which are responsive to gaseous or volatile liquid compounds since said compounds can rapidly be removed from the cell and its environment by stripping with gases like air. Recently, gas-inducible systems have been developed for fungal (US Pat. No. 5,710,021) or plant (US Pat. No. 6,380,463) cells, where gene expression can be adjusted in response to ketones or aldehydes including the volatile acetaldehyde (boiling point: 21°C) or to other compounds, which are metabolized to those regulating compounds, for example ethanol. The aforementioned gene regulation systems for fungal and plant cells are based on the *Aspergillus nidulans*-derived AlcR transcription factor, which, in the presence of acetaldehyde and related compounds, activates transcription from promoters containing specific operator sites derived or homologous to those found in the *A. nidulans* P_{alcA} promoter. In the absence of regulating compounds, AlcR-mediated transactivation is prevented and gene expression remains silent.

Chen (2002. Biochem.J. 368, 683-693) shows that acetaldehyde can activate the endogenous mammalian promoter for Tβ-RII. The promoter is activated by binding of AP-1 and BTEB. The binding is triggered by an unknown multistep mechanism via protein kinase C, ERK, JNK and other protein kinases. However, no evidence is provided that the same complex mechanism may work in mammalian cell lines other than hepatic stellate cells (HSC) possessing specific signal transduction elements.

White (Internet article, 1999-11-11 http://www.liv.ac.uk/-bates/MolBiol/ Projects98.html, retrieved on 2003-12-17) suggests to regulate luciferase expression in mammalian cells in response to ethanol by transfection of AlcR fused to a transcriptional activator together with a luciferase expression unit driven by an AlcA-derived promoter. However, this system has never been realized and, in the light of recent work, this system is not functional since ethanol is no direct inducer of the AlcR system (Flipphi, 2002. Biochem.J. 364, 25-3 1) and would rather require metabolization into acetaldehyde to be induction effective, which, in standard mammalian cell culture does not occur.

WO 97 06269 discloses a method for induction of herbicide resistance in plant cells by placing the herbicide resistance gene under control of an alcA-derived promoter, which is cotransfected with an expression vector for the acetaldehyde-responsive transactivator AlcR. Therefore, in the presence of ethanol, acetaldehyde is formed via alcohol dehydrogenase, which activates transcription from the alcA-derived promoter via interaction with AlcR.

### Summary of the Invention

The present invention is directed to mammalian cells and non-human mammals harboring transcription factors responsive to compounds being liquid or gaseous at the cultivation temperature of said mammalian cells or at the body temperature of said non-human mammals, and to mammalian cells and non-human mammals harboring the corresponding responsive promoters according to the claims. The invention is further directed to a method for adjusting gene expression in mammalian cells or non-human mammals by addition or removal of regulating compounds to the cell or mammal and its environment, whereby the regulating compounds are gaseous or liquid at the cultivation temperature of said cell or the body temperature of said non-human mammal according to the claims. In another embodiment, the invention is directed to isolated nucleic acids useful for construction of said mammalian cells according to the claims.

### Brief Description of the Drawings

Figure 1: Mode of function of the mammalian regulation system responsive to ketones or aldehydes like acetaldehyde.

Figure 1A: Expression vector for AlcR. The alcR gene (A) encoding the *Aspergillus nidulans*-derived AlcR protein is expressed under control of the simian virus 40-derived promoter (P _{SV40}). Transcription is terminated by a polyadenylation site (pA).

Figure 1B: The responsive promoter encodes an AlcR-specific operator site (OP) fused to a minimal promoter (Pₘᵢₙ) followed by a gene of interest (goi) and a polyadenylation site (pA).

Figure 1C: In the presence of gaseous or liquid regulating compounds (+RC), AlcR binds OP and activates Pₘᵢₙ resulting in expression of a gene of interest (goi).

Figure 1D: In the absence of regulating compounds (-RC), AlcR binding is prevented and expression of a gene of interest (goi) remains silent.

Figure 2: Gene expression in response to acetaldehyde in mammalian cells using the *Aspergillus nidulans*-dedved gene regulation system.

CHO-K1, BHK-21 and HeLa cells are transfected with the AlcR expression plasmid pWW195 and the corresponding promoter construct pWW192 driving expression of the reporter gene SEAP (human placental secreted alkaline phosphatase). Transfected cells are seeded into 96-well plates and acetaldehyde (24 µl/l) is applied to the first well of each row (well No. (WN) 1). Following incubation for 48 hours, relative SEAP production (in % relative to WN 1) is quantified in all wells.

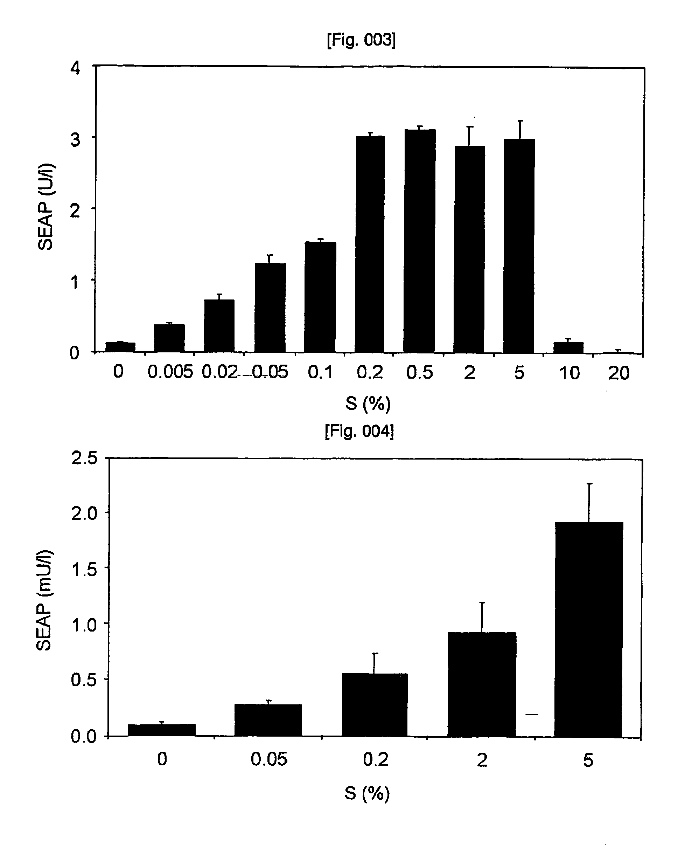

Figure 3: Gene expression in response to gaseous inducing compounds contained in tobacco smoke.

CHO-K1 cells are transfected with the AlcR expression plasmid pWW195 and the corresponding promoter construct pWW192 driving expression of the reporter gene SEAP (human placental secreted alkaline phosphatase). Transfected cells are incubated in atmospheres containing different concentrations of tobacco smoke (S, in % v/v) for 48 hours prior to quantification of SEAP production in the cell culture medium (in U/l: units per liter, 1 U corresponds to the hydrolysis of 1 µmol para-nitrophenylphosphate per min, Berger et al., 1998. Gene 66, 1-10).

Figure 4: Smoke-adjustable gene expression in mice.

CHO-K1 cells engineered with the AlcR expression plasmid pWW195 and the corresponding promoter construct pWW192 driving expression of the reporter gene secreted alkaline phosphatase (SEAP) are micro-encapsulated in alginate-poly-L-lysine-alginate capsules and intraperitoneally implanted into mice. Mice are exposed to different tobacco smoke concentrations (S, in % v/v) for 72 hours with smoke renewal every 12 hours prior to quantification of SEAP serum levels in mice (in mU/l).

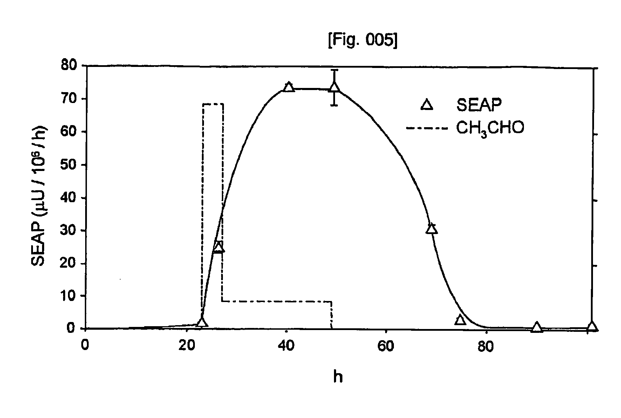

Figure 5: Gas-phase controlled gene expression in a bioreactor.

A stable CHO-K1-derivative engineered for acetaldehyde-inducible SEAP expression is cultivated in a bioreactor. Acetaldehyde (CH CHO) is applied by sparging the culture medium with air containing the indicated acetaldehyde concentrations (ppm). The specific SEAP productivities (µU/10⁶ cells/h) are indicated over time (h).

### Detailed description of the invention

For the purpose of the invention, a regulating compound (RC) is any compound or compound mix, which is gaseous or liquid at cultivation temperature of mammalian cells or at the body temperature of non-human mammals, and which directly or indirectly interacts with a responsive transcription factor (RTF) and thereby modulates transactivation activity of promoters harboring at least one OP operator site, to which said RTF can bind. The RC can be, as non-limited examples, selected from one of the following groups: ketones, aldehydes, alkanes, balcalkanes, alcohols, esters, amines and ethers like, for example and without limitation, ethanol, methylamine, ethylamine, n-propylamine, n-butylamine, n-pentylamine, n-hexylamine, benzylamine, n-propanol, n-butanol, 2-propanol, 2-butanol, 2-methylbutyraldehyde, acetaldehyde, propanal, acetone, 2-butanone, 2-pentanone, 3-pentanone, cyclohexanone, glycoaldehyde, glyoxal, glyoxylate, ethylene glycol, ethanolamine, ethyl acetate, ethyl ether, and dicyclopropylketone, and compounds that are metabolized *in situ* to such RCs, e.g. L-threonine or glycine.

For the purpose of the invention, a responsive transcription factor (RTF) is any polypeptide, which modulates transcription activity of OP-containing promoters in response to direct or indirect interaction with an RC molecule. The modulation of transcription activity mediated by RTF in response to RC is either caused by altered binding characteristics of RTF to OP-containing promoters or by interaction of RC with RTF in a way that RTF alters its transactivation potential, for example via conformational changes. The RTF protein may comprise proteins identical to, derived from or related to proteins naturally encoded by prokaryotes or eukaryotes, either on the chromosome or an episome. Preferably the RTF protein comprises proteins identical to, derived from or related to proteins naturally encoded by heterologous hosts of non-mammalian origin.

For the purpose of the invention, the operator sequence (OP) may comprise a naturally occurring polynucleotide sequence or a polynucleotide sequence derived from a naturally occurring polynucleotide sequence, which can be bound by an RTF either constitutively or in an RC-dependent way.

By an RTF comprising proteins being "derived from" naturally occurring proteins is meant, in this context, that the RTF comprises protein domains that contain amino acid substitutions, preferably conservative amino acid substitutions, but remain at least 70%, preferably 80%, and more preferably 90% or more identical to the naturally occurring proteins at the amino acid level.

By an RTF comprising proteins "related to" naturally occurring proteins is meant, for purposes of the invention, that the polynucleotide sequence which encodes the amino acid sequence of the RTF protein hybridizes to a naturally occurring polynucleotide sequence encoding a naturally occurring protein under at least low stringency conditions, more preferably moderate stringency conditions, and most preferably high stringency conditions.

"Conservative substitution" is known in the art and is described e.g. by Dayhof, M. D.,1978, Nat. Biomed Res Found., Washington, D.C., Vol. 5, Sup. 3. Genetically encoded amino acids are generally divided into four groups: (1) acidic = aspartate and glutamate; (2) basic = lysine, arginine, and histidine; (3) non-polar = alanine, valine, leucine, isoleucine, proline, phenylalanine, methionine, and tryptophan; and (4) uncharged polar = glycine, asparagine, glutamine, cysteine, serine, threonine, and tyrosine. Phenylalanine, tryptophan and tyrosine are also jointly classified as aromatic amino acids. A substitution in a protein of one amino acid classified in a particular group by another amino acid in the same group is generally regarded as a conservative substitution.

By an operator sequence (OP) "derived from" a naturally occurring polynucleotide sequence is meant, in this context, that the polynucleotide sequence of the OP contains base changes or modified nucleotides compared to the naturally occurring polynucleotide, but still can bind the RTF either constitutively or in an RC-dependent way.

The present invention is directed to mammalian cells comprising an RTF and a corresponding OP binding partner, which may be functionally linked to promoters or fragments thereof. More specifically the invention relates to such a mammalian cell further comprising a nucleic acid (i.e. a gene of interest) encoding a desired protein functionally linked to the promoter or promoter fragments.

Particular pairs of RTF and OP binding partners are, for example, the *Aspergillus nidulans* AlcR protein (Flipphi et al., 2002. Biochem J 364, 25-31), which binds to the corresponding OP operator sequence (for example Genbank Accession No. S47331, nucleotides 30-308) in response to the addition of RC molecules as mentioned hereinbefore and to those described in Flipphi et al. (2002, Biochem J 364, 25-31) and the references therein; and the *Pseudomonas putida (oleovorans)* AlkS protein (Smits et al., 2001. Plasmids 46, 16-24), which binds to its corresponding OP binding partner derived from the P_{alkB} promoter in response to RC molecules as described hereinbefore.

An RTF protein and the specific OP binding partner 8 may be found, for example by sequence database searching for proteins derived from or related to known RTF proteins using e.g. a BLAST (Altschul et al., 1997, Nucleic Acids Res. 25, 3389-402) computer program.

The RTF may be an assembly of different protein domains, which may comprise either artificially designed sequences or sequences derived from or related to naturally occurring sequences. In particular, the RTF may comprise protein domains which either activate or repress transcription of promoters functionally linked to OP binding sites. Activating domains useful for the present invention are, for example, the Herpes simplex VP16 domain or minimal versions thereof, and activating domains derived from or related to GAL4, p65, CTF/NF1, AP2, ITP1, ITF2, Oct1 and Sp1. Repressing protein domains may be derived from or related to, for example, the v-erbA oncogene product, the thyroid hormone receptor, the *Drosophila* Krueppel protein, the KRAB protein domain, the Ssn6/Tup1 protein complex, the SIRI protein, NeP1, TSF3, SF1, WT1, Oct-2.1, E4BP4 and ZF5. Other repressing or activating polypeptides as listed in US Pat. No. 6,287,813 and those to be found according to the references cited therein are also within the scope of this invention.

In another preferred embodiment the invention is directed to non-human mammals comprising mammalian cells containing an RTF and a corresponding OP binding partner, which may be functionally linked to promoters or fragments thereof.

Described is a method of using a mammalian cell or a non-human mammal containing an RTF, and the corresponding OP binding partner for modulating gene expression in those organisms in response to the exogenous or endogenous administration of RC molecules. Therefore, the gene of interest may be fused downstream of a naturally occurring or artificial promoter or a promoter fragment containing at least one OP site. This genetic construct is transferred into a mammalian cell or a non-human mammal also containing an expression vector for an RTF protein. The method comprises administration of RC molecules either via the gas phase or directly into the cultivation medium or the mammal in liquid form resulting in a modulated transactivation potential of RTF or in a changed interaction between RTF and OP leading to changed expression levels of the gene of interest. Additionally, the RC can be administered indirectly as a precursor compound, which is metabolized *in situ* to the active RC form.

In particular the invention relates to a method for adjusting the expression level of a protein in a mammalian cell as described hereinbefore comprising culturing said mammalian cell and modulating gene expression by administration of a compound for which transcription of the OP operator-containing promoter and the responsive transcription factor RTF are responsive.

Furthermore the invention relates to a method for adjusting the expression level of a gene in a mammalian cell as described hereinbefore, comprising
1. functionally linking said gene to a OP-containing promoter,
2. transferring said OP-containing promoter functionally linked to said gene into said mammalian cell, and
3. inducing expression of said gene by activating said OP-containing promoter by administration of a compound for which the OP operator-specific responsive transcription factor RTF is responsive.

The gene of interest may be any artificial or naturally occurring gene, either endogenous or exogenous to the mammalian cell, and may be, for example, the gene encoding SEAP, a fluorescent protein, human growth hormone, alpha-interferon, beta-interferon, gamma-interferon, insulin, erythropoietin, tissue plasminogen activator, DNAse, a monoclonal antibody, Factor VIII, Factor VII, HAS, IL-2, glucagons, EGF, GCSF, GMCSF, thrombopoietin, gp160, HbSAg, or any tumor suppressor gene. The gene product can also be any protein interfering with absorption, distribution, metabolism or excretion of compounds contained in tobacco smoke, like nicotine.

The invention is further directed to isolated nucleic acids for expression of RTF in mammalian cells, said nucleic acids comprising a sequence coding for RTF under control of a constitutive or regulatable promoter functional in mammalian cells, and to isolated nucleic acids containing OP sequences functionally linked to promoters or promoter fragments functional in mammalian cells. The isolated nucleic acids may further comprise sequences useful for constructing viral vectors, like, for example, those based on or related to adenoviruses, adeno-associated viruses, retroviruses, alphaviruses, papillomaviruses, and picormaviruses.

The invention having been described, the following examples are offered bv wav of illustration

Example 1: Construction of a gas-inducible expression system for mammalian cells.

A gas-inducible expression system for mammalian cells is constructed based on the *Aspergillus nidulans* AlcR-P_{AlcA} interaction responsive to small molecular compounds of the aldehyde and ketone class like acetaldehyde, which are gaseous at mammalian cell cultivation temperature (boiling point: 21°C) or at the body temperature of non-human mammals comprising those mammalian cells.

The responsive transcription factor AlcR is cloned under control of the simian virus 40 promoter (P_{SV40}) by excising alcR (EcoRI/SalI) from an alcR cDNA containing vector (Flipphi et al., 2002. Biochem J 364, 25-31) and cloning it (EcoRI/XhoI) into pWW75 (Weber et al., 2002. Biotechnol Bioeng 80,691-705), thereby resulting in plasmid pWW195.

A responsive promoter is designed by cloning the AlcR-specific OP site derived from the *Aspergillus nidulans* P_{alcA} promoter 5' of a minimal version of the human cytomegalovirus immediate early promoter (US Pat. No. 5,464,758), which controls expression of the human placental secreted alkaline phosphatase SEAP. The OP site is PCR-amplified from a P_{AlcA} containing vector (Genbank Accession No. S47331) using oligonucleotides OWW58 (5'-gatcgacgtcggagctaccatccaataaccc-3') and OWW59 (5'-gatccctgcaggcccgctcgtttgtggctct-3') and cloned (AatII/SbfI) into pWW37 (Weber et al., 2002. Biotechnol Bioeng 80, 691-705), thereby resulting in plasmid pWW192.

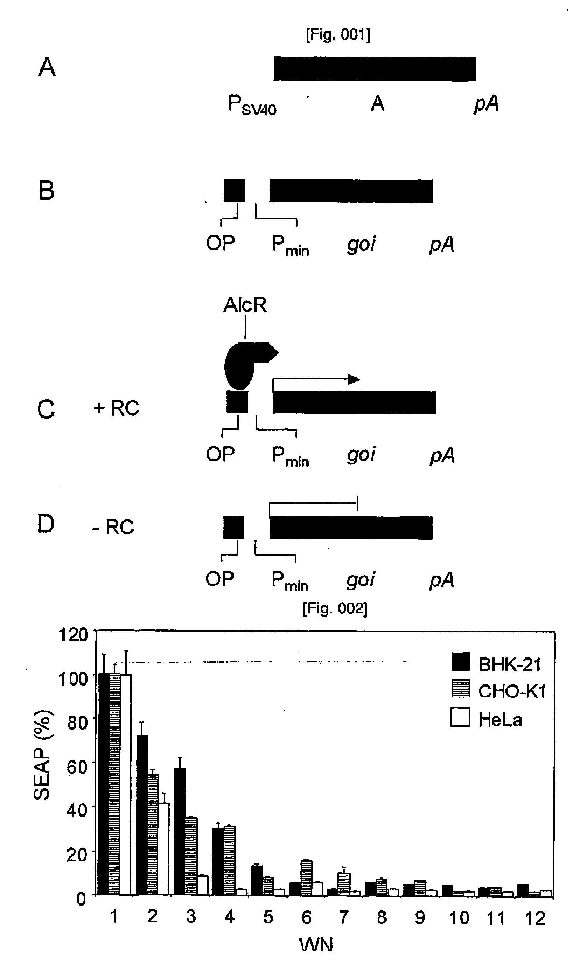

These constructs are used for gas-inducible expression of the reporter gene SEAP in Chinese hamster ovary cells (CHO-K1), baby hamster kidney cells (BHK-21) and human HeLa cells. These cell lines are seeded in 96-well plates (3 rows per cell line) and cotransfected with plasmids pWW192 and pWW195 using standard calcium phosphate methods. The first wells in a row (WN 1) are supplemented with 24 µl/l acetaldehyde, and the plates are incubated in a humidified 5% CO₂-containing atmosphere for 48 hours prior to quantification of SEAP production in all wells using a chemiluminescence kit from Roche (Roche Applied Science, Rotkreuz, Switzerland). The relative SEAP production in each well is shown in Figure 2. SEAP production is highest in acetaldehyde-containing wells and decreased in a distance-dependent manner for all three cell lines. Those results demonstrate, that the chimeric expression system based on *Aspergillus nidulans* genetic elements is (i) functional in different mammalian cell lines including human ones, (ii) enables inducible gene expression by addition of a liquid inducer acetaldehyde, and (iii) enables inducible gene expression by the gaseous inducer acetaldehyde, which diffuses through the gas phase to neighboring wells where it induces gene expression.

Example 2: Regulatable gene expression in mammalian cells induced by a complex gas mixture.

Acetaldehyde and related compounds are prevalent in complex gas mixtures like, for example, tobacco smoke. Tobacco smoke can be used to induce gene expression in mammalian cells harboring the genetic elements for gas-adjustable gene expression as described in Example 1.

CHO-K1 cells are transfected with plasmids pWW192 and pWW195 and exposed to different tobacco smoke concentrations in the culture atmosphere for 48 hours prior to quantification of SEAP production (Figure 3): SEAP expression gradually increases between 0.005% (v/v) smoke (0.06 ppm acetaldehyde) and 0.2% (v/v) smoke (2.4 ppm acetaldehyde), reaches a maximum expression plateau between 0.2% and 5% (v/v) smoke (60 ppm acetaldehyde) and collapses beyond 5% (v/v) tobacco smoke due to cell death associated with the undefined cytotoxic compound cocktail contained in tobacco smoke.

Example 3: Gas-inducible gene expression in animals.

The relative low toxicity of acetaldehyde (No Observable Effect Level: 152 ppm) predestinates this system for adjusting gene expression in non-human mammals in prototype gene therapy settings. CHO-K1 cells engineered for acetaldehyde-responsive gene expression (Example 1) are microencapsulated into alginate-poly-L-lysine-alginate microspheres using a droplet generator set to produce spheres of 400±50 µm in diameter, with each sphere containing 50-400 cells. 700 µl of capsule suspension (50% v/v) in MOPS-buffered physiological salt solution are intraperitoneally injected into mice, and the mice kept in 1001 high-density polyethylene boxes (8 mice per box) sealed with paraffin. The boxes are filled with different tobacco smoke concentrations (smoke and air exchange in the boxes every 12 hours). After 72 hours serum is collected from the mice and activity of the reporter enzyme SEAP quantified using a chemiluminescence kit. The different SEAP levels (Figure 4) increase with increasing smoke concentrations and therefore show, that gas-inducible gene expression systems are able to titrate desired proteins in non-human mammals to specific levels.

Example 4: Viral vectors for transduction of mammalian cells with the gas-responsive gene expression system.

A retroviral vector is constructed for easy transduction of mammalian cells and non-human mammals with the acetaldehyde-responsive transcription factor AlcR. AlcR excised (EcoRI/SalI) from pAlcR is cloned (EcoRI/XhoI) into the pMSCV-derived (Mouse Stem Cell Virus, Clontech, Palo Alto, CA) vector pWW100 (Weber et al., 2002. Biotechnol Bioeng 80, 691-705) thereby resulting in vector pWW506. Retroviral particles are constructed by transient transfection of GP-293 cells (Clontech, Palo Alto, CA) with pWW506 according to the manufacturer's recommendations.

The responsive promoter containing the AlcR-specific OP site is PCR-amplified from plasmid pWW192 (Example 1) and ligated into the 3'-LTR sequence of a LTR_{ΔU3} third-generation lentiviral vector (Mitta et al., 2002. Nucleic Acids Res. 30, e113). The lentiviral vector also contains a multiple cloning site for inserting any gene of interest and a neomycin-resistance gene for selection of transduced cells. Cotransfection of the vector construct together with the helper-plasmids pLTR-G and pCD/NL-BH* (Mitta et al., 2002. Nucleic Acids Res. 30, e113) into HEK 293-T cells produces viral particles, which are harvested from the supernatant and filtrated through a 0.45 µm low protein-binding filter. The viral particles can be readily used for transduction of any mammalian target cell or can be stored at -80°C.

Example 5: Gas-inducible gene expression in mammalian cells based on prokaryotic regulator proteins.

A regulatable expression system responsive to liquid or gaseous inducers based on bacterial elements is constructed by PCR-cloning the *Pseudomonas putida (oleovorans)*-derived RTF AlkS (Smiths et al., 2001. Plasmid 46, 16-24) into the mammalian expression vector pEF6/V5-His TOPO (Invitrogen, Carlsbad, CA). The responsive promoter is constructed by cloning 8 repeats of the AlkS-specific OP site downstream of the simian virus 40 promoter P_{SV40} followed by a reporter gene, the secreted alkaline phosphatase SEAP.

Transfection of the plasmids encoding AlkS and the responsive promoter controlling SEAP expression in CHO-K1 cells results in SEAP expression levels typical for P_{SV40}-driven constitutive expression. However, upon addition of a cognate regulating compound (RC), like dicyclopropylketone, haloalkanes, ethyl acetate or ethyl ether, SEAP expression decreases in a dose-dependent manner, due to an RC-mediated interaction of AlkS with its cognate OP site leading to sterical hindrance of transcription.

Example 6: Gas-phase mediated reversible gene expre ssion in a bioreactor.

A CHO-K1-derived cell line is constructed for gas-inducible expression of a secreted alkaline phosphatase (SEAP) by stable transfection/transduction with plasmid pWW192 (see Example 1) and a pWW506-based viral particle (see Example 4). The cell line is cultivated in a 2.5 L bioreactor, and SEAP expression is induced by gassing in air containing 340 ppm acetaldehyde, followed by a constant expression phase during which the air inflow contains 40 ppm acetaldehyde. Finally gene expression is shut down by stripping out acetaldehyde contained in the reactor by sparging the medium with pure air (Figure 5). This example demonstrates that acetaldehyde-responsive expression technology can be used in bioreactors for inducing and reversing gene expression via sparging the culture medium with air.

## Claims

1. A mammalian cell comprising
a. a responsive transcription factor RTF, selected from *Aspergillus nidulans* AlcR and proteins comprising amino acid sequences being 80% or more identical to AlcR, which modulates transcription of promoters containing an operator sequence, which can bind the RTF, in response to acetaldehyde; or a responsive transcription factor RTF selected from *Pseudomonas putida* AlkS and proteins comprising amino acid sequences being 80% or more identical to AlkS, which modulates transcription of promoters containing an operator sequence, which can bind the RTF, in response to compounds being gaseous or liquid at the cultivation temperature of said mammalian cell selected from ketones, aldehydes, alkanes, haloalkanes, alcohols, esters, amines, ethers and compounds that are metabolized *in situ* to these classes of compounds;
b. a promoter or promoter fragments operatively linked to operator sequence sites specific for binding of said RTF.

2. The mammalian cell of claim 1 further comprising a nucleic acid encoding a desired protein functionally linked to said promoter or promoter fragments operatively linked to operator sequence sites specific for binding said RTF.

3. The mammalian cell of claim 1 or 2, wherein binding of the RTF to said operator sequence containing promoters is changed in response to compounds being gaseous at the cultivation temperature of said mammalian cell.

4. The mammalian cell of claim 1 or 2, wherein binding of the RTF to said operator sequence containing promoters is changed in response to compounds being liquid at the cultivation temperature of said mammalian cell.

5. The mammalian cell of claim 1, wherein the RTF comprises amino acid sequences being 90% or more identical to AlcR or AlkS.

6. The mammalian cell of claim 1 or 2, wherein the RTF is the *Aspergillus nidulans* AlcR protein.

7. The mammalian cell of claim 1 or 2, wherein the RTF is the *Pseudomonas putida* AlkS protein.

8. A non-human mammal comprising at least one mammalian cell as claimed in claim 1 or 2.

9. An in vitro method for adjusting the expression level of a desired protein in a mammalian cell as claimed in claim 2, comprising culturing said mammalian cell and modulating gene expression by administration of a compound, for which transcription of the operator sequence containing promoter and the responsive transcription factor RTF are responsive, selected from
acetaldehyde if the RTF is *Aspergillus nidulans* AlcR or a protein comprising amino acid sequences being 80% or more identical to AlcR, or
ketones, aldehydes, alkanes, haloalkanes, alcohols, esters, amines, ethers and compounds that are metabolized *in situ* to these classes of compounds if the RTF is *Pseudomonas putida* AlkS or a protein comprising amino acid sequences being 80% or more identical to AlkS.

10. The method of claim 9, wherein the desired protein for which the expression level is adjusted is selected from the group consisting of SEAP, a fluorescent protein, human growth hormone, alpha-interferon, beta-interferon, gamma-interferon, insulin, erythropoietin, tissue plasminogen activator, DNAse, a monoclonal antibody. Factor VIII, Factor VII, HAS, IL-2. glucagons. EGF, GCSF, GMCSF, thrombopoietin, gp160, HbSAg, and a protein encoded by a tumor suppressor gene.

11. The method of claim 9 or 10, wherein the RTF is *Aspergillus nidulans* AlcR or a protein comprising amino acid sequences being 80% or more identical to AlcR and the compound for modulating gene expression is acetaldehyde.

12. The method of claim 9 or 10, wherein the RTF is *Pseudomonas putida* AlkS or a protein comprising amino acid sequences being 80% or more identical to AlkS and the compound for modulating gene expression is selected from the group consisting of ethanol, methylamine, ethylamine, n-propylamine, n-butylamine, n-pentylamine, n-hexylamine, benzylamine, n-propanol, n-butanol, 2-propanol, 2-butanol, 2-methylbutyraldehyde, acetaldehyde, propanal, acetone, 2-butanone, 2-pentanone, 3-pentanone, cyclohexanone, glycoaldehyde, glyoxal, glyoxylate, ethylene glycol, ethanolamine, ethyl acetate, ethyl ether, dicyclopropylketone, and compounds that are metabolized *in situ* to said compounds.

13. The method of claim 9 or 10, wherein the RTF is the *Aspergillus nidulans* AlcR protein and the compound for modulating gene expression is acetaldehyde.

14. An in vitro method for adjusting the expression level of a gene in a mammalian cell as claimed in claim 1, comprising
a. functionally linking said gene to an operator sequence containing promoter, the transcription of which is modulated by the responsive transcription factor RTF,
b. transferring said operator sequence containing promoter functionally linked to said gene into said mammalian cell, and
c. modulating expression of said gene by activating said operator sequence containing promoter by administration of a compound for which the operator sequence specific responsive transcription factor RTF is responsive.

15. The method of claim 14, wherein the gene codes for a protein selected from the group consisting of SEAP, a fluorescent protein, human growth hormone, alpha-interferon, beta-interferon, gamma-interferon, insulin, erythropoietin, tissue plasminogen activator, DNAse, a monoclonal antibody, Factor VIII, Factor VII, HAS, IL-2, glucagons, EGF, GCSF, GMCSF, thrombopoietin, gp160, and HbSAg.

16. The method of claim 14, wherein the gene is a tumor suppressor gene.

17. The method of anyone of claims 14 to 16, wherein the RTF is *Aspergillus nidulans* AlcR or a protein comprising amino acid sequences being 80% or more identical to AlcR and the compound for which the operator sequence-specific responsive transcription factor RTF is responsive is acetaldehyde.

18. The method of anyone of claims 14 to 16, wherein the RTF is *Pseudomonas putida* AlkS or a protein comprising amino acid sequences being 80% or more identical to AlkS and the compound for which the operator sequence-specific responsive transcription factor RTF is responsive is selected from the group consisting of ethanol, methylamine, ethylamine, n-propylamine, n-butylamine, n-pentylamine, n-hexylamine, benzylamine, n-propanol, n-butanol, 2-propanol, 2-butanol, 2-methylbutyraldehyde, acetaldehyde, propanal, acetone, 2-butanone, 2-pentanone, 3-pentanone, cyclohexanone, glycoaldehyde, glyoxal, glyoxylate, ethylene glycol, ethanolamine, ethyl acetate, ethyl ether, dicyclopropylketone, and compounds that are metabolized *in situ* to said compounds.

19. The method of claim 14 wherein the operator sequence containing promoter is an AlcR-specific operator sequence site, RTF is the *Aspergillus nidulans* AlcR protein, an the compound for which RTF is responsive is acetaldehyde.

20. An isolated nucleic acid useful for constructing a mammalian cell as claimed in claim 1, comprising an RTF-encoding nucleic acid, wherein the RTF is selected from *Aspergillus nidulans* AlcR, *Pseudomonas putida* AlkS, and proteins comprising amino acid sequences being 80% or more identical to AlcR and AlkS. functionally linked to a promoter useful for expression of the RTF in said mammalian cell.

21. The isolated nucleic acid of claim 20 comprising an operator sequence functionally linked to a promoter or a fragment thereof useful for RTF-dependent gene expression in said mammalian cell.

22. The isolated nucleic acid of claim 20 or 21 further comprising genetic elements useful for construction of viral vectors.

## Patentansprüche

1. Säugerzelle umfassend
a. einen ansprechbaren Transkriptionsfaktor RTF, ausgewählt aus *Aspergillus nidulans* AlcR und Proteinen, die mit AlcR zu 80% oder mehr identische Aminosäuresequenzen umfassen, welcher die Transkription von Promotoren enthaltend eine Operatorsequenz moduliert, die den RTF als Antwort auf Acetaldehyd binden kann; oder einen ansprechbaren Transkriptionsfaktor RTF, ausgewählt aus *Pseudomonas putida* alkS und Proteinen, die mit AlkS zu 80% oder mehr identische Aminosäuresequenzen umfassen, welcher die Transkription von Promotoren enthaltend eine Operatorsequenz moduliert, die den RTF als Antwort auf bei der Kultivierungstemperatur besagter Säugerzellen gasförmige oder flüssige Verbindungen binden können, ausgewählt aus Ketonen, Aldehyden, Alkanen, Haloalkanen, Alkoholen, Estern, Aminen, Ethern und Verbindungen, die *in situ* zu diesen Verbindungsklassen metabolisiert werden; und
b. einen Promotor oder Promotorenfragmente, operativ an die Operatorsequenz-Regionen gebunden, die spezifisch für die Bindung besagter RTF sind.

2. Säugerzelle gemäss Anspruch 1, die weiter eine Nukleinsäure umfasst, die für ein gewünschtes Protein kodiert, und funktionell an besagten Promotor oder Promotorenfragmente gebunden ist, die operativ an die Operatorsequenz-Regionen gebunden sind, die spezifisch für die Bindung besagter RTF sind.

3. Säugerzelle gemäss Anspruch 1 oder 2, worin die Bindung des RTF an Promotoren enthaltend besagte Operatorsequenz als Antwort auf bei der Kultivierungstemperatur besagter Säugerzellen gasförmige Verbindungen geändert wird.

4. Säugerzelle gemäss Anspruch 1 oder 2, worin die Bindung des RTF an Promotoren enthaltend besagte Operatorsequenz als Antwort auf bei der Kultivierungstemperatur besagter Säugerzellen flüssige Verbindungen geändert wird.

5. Säugerzelle gemäss Anspruch 1, worin der RTF mit AlcR oder AlkS zu 90% oder mehr identische Aminosäuresequenzen umfasst.

6. Säugerzelle gemäss Anspruch 1 oder 2, worin der RTF das *Aspergillus nidulans* AlcR Protein ist.

7. Säugerzelle gemäss Anspruch 1 oder 2, worin der RTF das *Pseudomonas putida* AlkS Protein ist.

8. Ein nicht-menschlicher Säuger umfassend wenigstens eine Säugerzelle wie in Anspruch 1 oder 2 beansprucht.

9. *In vitro* Verfahren zur Anpassung des Expressionsgrades eines gewünschten Proteins in einer wie in Anspruch 2 beanspruchten Säugerzelle, umfassend die Kultivierung besagter Säugerzelle und Modulierung der Gen-Expression durch Verabreichung einer Verbindung, auf welche die Transkription des die Operatorsequenz enthaltenden Promotors und der ansprechbare Transkriptionsfaktor RTF ansprechen, ausgewählt aus
Acetaldehyd, falls der RTF ein *Aspergillus nidulans* AlcR oder ein Protein umfassend mit AlcR zu 80% oder mehr identische Aminosäurensequenzen ist, oder Ketonen, Aldehyden, Alkanen, Haloalkanen, Alkoholen, Estern, Aminen, Ethern und Verbindungen, die *in situ* zu diesen Verbindungsklassen metabolisiert werden, falls der RTF ein *Pseudomonas putida* AlkS oder ein Protein umfassend mit AlkS zu 80% oder mehr identische Aminosäurensequenzen ist.

10. Verfahren gemäss Anspruch 9, worin das gewünschte Protein, für das der Expressionsgrad angepasst wird, aus der Gruppe bestehend aus SEAP, einem fluoreszierenden Protein, menschlichem Wachstumshormon, Interferon alpha, Interferon beta, Interferon gamma, Insulin, Erythropoietin, Gewebeplasminogen-Aktivator, Desoxyribonuklease, einem monoklonalen Antikörper, Faktor VIII, Faktor VII, HAS, IL-2, Glucagonen, EGF, GCSF, GMCSF, Thrombopoietin, gp160, HbSAg und einem Protein, das durch ein Tumorsupressor-Gen kodiert wird, ausgewählt ist.

11. Verfahren gemäss Anspruch 9 oder 10, worin der RTF *Aspergillus nidulans* AlcR oder ein Protein ist, das mit AlcR zu 80% oder mehr identische Aminosäuresequenzen umfasst, und die die Gen-Expression modulierende Verbindung Acetaldehyd ist.

12. Verfahren gemäss Anspruch 9 oder 10, worin der RTF *Pseudomonas putida* AlkS oder ein Protein ist, das mit AlkS zu 80% oder mehr identische Aminosäuresequenzen umfasst, und die die Gen-Expression modulierende Verbindung aus der Gruppe bestehend aus Ethanol, Methylamin, Ethylamin, n-Propylamin, n-Butylamin, n-Pentylamin, n-Hexylamin, Benzylamin, n-Propanol, n-Butanol, 2-Propanol, 2-Butanol, 2-Methylbutyraldehyd, Acetaldehyd, Propanal, Aceton, 2-Butanon, 2-Pentanon, 3-Pentanon, Cyclohexanon, Glycoaldehyd, Glyoxal, Glyoxylat, Ethylenglycol, Ethanolamin, Ethylacetat, Ethylether, Dicyclopropylketon und Verbindungen, die *in situ* zu besagten Verbindungen metabolisieren, ausgewählt ist.

13. Verfahren gemäss Anspruch 9 oder 10, worin der RTF *Aspergillus nidulans* A1cR Protein und die die Gen-Expression modulierende Verbindung Acetaldehyd ist.

14. *In vitro* Verfahren zur Anpassung des Expressionsgrades eines Gens in einer Säugerzelle wie in Anspruch 1 beansprucht, umfassend
a. funktionelle Verknüpfung des besagten Gens mit einem eine Operatorsequenz enthaltenden Promotor, dessen Transkription durch den ansprechbaren Transkriptionsfaktor RTF moduliert wird,
b. Transfer des besagte Operatorsequenz enthaltenden Promotors, der funktionell mit besagtem Gen verknüpft ist, in besagte Säugerzelle, und
c. Modulierung der Expression des besagten Gens durch Aktivierung des besagte Operatorsequenz enthaltenden Promotors durch Verabreichung einer Verbindung, auf die der auf die Operatorsequenz spezifisch ansprechbare Transkriptionsfaktor RTF anspricht.

15. Verfahren gemäss Anspruch 14, worin das Gen für ein Protein ausgewählt aus der Gruppe bestehend aus SEAP, einem fluoreszierenden Protein, menschlichem Wachstumshormon, Interferon alpha, Interferon beta, Interferon gamma, Insulin, Erythropoietin, Gewebeplasminogen-Aktivator, Desoxyribonuklease, einem monoklonalen Antikörper, Faktor VIII, Faktor VII, HAS, IL-2, Glucagonen, EGF, GCSF, GMCSF, Thrombopoietin, gp160 und HbSAg kodiert.

16. Verfahren gemäss Anspruch 14, worin das Gen ein Tumorsuppressor-Gen ist.

17. Verfahren gemäss irgendeinem der Ansprüche 14 bis 16, worin der RTF *Aspergillus nidulans* AlcR oder ein Protein ist, das mit AlcR zu 80% oder mehr identische Aminosäuresequenzen umfasst, und die Verbindung, auf welche der auf die Operatorsequenz spezifisch ansprechbare Transkriptionsfaktor RTF anspricht, Acetaldehyd ist.

18. Verfahren gemäss irgendeinem der Ansprüche 14 bis 16, worin der RTF *Pseudomonas putida* AlkS oder ein Protein ist, das mit AlkS zu 80% oder mehr identische Aminosäuresequenzen umfasst, und die Verbindung, auf welche der auf die Operatorsequenz spezifisch ansprechbare Transkriptionsfaktor RTF anspricht, aus der Gruppe bestehend aus Ethanol, Methylamin, Ethylamin, n-Propylamin, n-Butylamin, n-Pentylamin, n-Hexylamin, Benzylamin, n-Propanol, n-Butanol, 2-Propanol, 2-Butanol, 2-Methylbutyraldehyd, Acetaldehyd, Propanal, Aceton, 2-Butanon, 2-Pentanon, 3-Pentanon, Cyclohexanon, Glycoaldehyd, Glyoxal, Glyoxylat, Ethylenglycol, Ethanolamin, Ethylacetat, Ethylether, Dicyclopropylketon und Verbindungen, die *in situ* zu besagten Verbindungen metabolisieren, ausgewählt ist.

19. Verfahren gemäss Anspruch 14, worin der eine Operatorsequenz enthaltende Promotor eine auf AlcR spezifische Operatorsequenz-Region, RTF das *Aspergillus nidulans* AlcR Protein und die Verbindung, auf die der RTF reagiert, Acetaldehyd ist.

20. Isolierte, für die Herstellung einer wie in Anspruch 1 beanspruchten Säugerzelle nützliche Nukleinsäure, umfassend eine für RTF kodierende Nukleinsäure, worin der RTF aus *Aspergillus nidulans* AlcR, *Pseudomonas putida* AlkS und Proteinen, die mit AlcR und AlkS zu 80% oder mehr identische Aminosäuresequenzen umfassen, ausgewählt ist, und die funktionell mit einem für die Expression von RTF in besagter Säugerzelle nützlichen Promotor verknüpft ist.

21. Isolierte Nukleinsäure gemäss Anspruch 20 umfassend eine Operatorsequenz, die funktionell mit einem Promotor oder einem Fragment davon verknüpft ist, das für die von RTF abhängige Gen-Expression in besagter Säugerzelle nützlich ist.

22. Isolierte Nukleinsäure gemäss Anspruch 20 oder 21, die weiter für die Herstellung viraler Vektoren nützliche genetische Elemente umfasst.

## Revendications

1. Cellule de mammifère comprenant :
a. un facteur de transcription sensible RTF, choisi parmi l'AlcR d'*Aspergillus nidulans* et des protéines comprenant des séquences d'acides aminés qui sont identiques à 80% ou plus à l'AlcR, lequel module la transcription de promoteurs contenant une séquence opérateur, qui peut se lier au RTF, en réponse à l'acétaldéhyde ; ou
un facteur de transcription sensible RTF, choisi parmi l'AlkS de *Pseudomonas putida* et des protéines comprenant des séquences d'acides aminés qui sont identiques à 80% ou plus à l'AlkS, lequel module la transcription de promoteurs contenant une séquence opérateur, qui peut se lier au RTF, en réponse à des composés étant gazeux ou liquides à la température de culture de ladite cellule de mammifère, choisis parmi les cétones, aldéhydes, alcanes, halo-alcanes, alcools, esters, amines, éthers et des composés qui sont métabolisés *in situ* en ces classes de composés ;
b. un promoteur ou des fragments de promoteur lié(s), de manière opérationnelle, à des sites de séquences opérateurs spécifiques de la liaison audit RTF.

2. Cellule de mammifère selon la revendication 1, comprenant en outre un acide nucléique codant pour une protéine souhaitée, qui est lié, de manière fonctionnelle, audit promoteur ou auxdits fragments de promoteur lié(s), de manière opérationnelle, à des sites de séquences opérateurs spécifiques de la liaison audit RTF.

3. Cellule de mammifère selon les revendications 1 ou 2, dans laquelle la liaison du RTF auxdits promoteurs contenant une séquence opérateur est changée en réponse à des composés qui sont gazeux à la température de culture de ladite cellule de mammifère.

4. Cellule de mammifère selon les revendications 1 ou 2, dans laquelle la liaison du RTF auxdits promoteurs contenant une séquence opérateur est changée en réponse à des composés qui sont liquides à la température de culture de ladite cellule de mammifère.

5. Cellule de mammifère selon la revendication 1, dans laquelle le RTF comprend des séquences d'acides aminés étant identiques à 90% ou plus à l'AlcR ou à l'AlkS.

6. Cellule de mammifère selon les revendications 1 ou 2, dans laquelle le RTF est la protéine AlcR *d'Aspergillus nidulans.*

7. Cellule de mammifère selon les revendications 1 ou 2, dans laquelle le RTF est la protéine AlkS de *Pseudomonas putida.*

8. Mammifère non humain comprenant au moins une cellule de mammifère selon les revendications 1 ou 2.

9. Procédé *in vitro* destiné à ajuster le niveau d'expression d'une protéine souhaitée dans une cellule de mammifère, selon la revendication 2, comprenant les étapes consistant à cultiver ladite cellule de mammifère et à moduler l'expression génique, en administrant un composé auquel sont sensibles la transcription du promoteur contenant une séquences opérateur et le facteur de transcription sensible RTF, choisi parmi :
l'acétaldéhyde, si le RTF est l'AlcR *d'Aspergillus nidulans* ou une protéine comprenant des séquences d'acides aminés qui sont identiques à 80% ou plus à l'AlcR ; ou
des cétones, aldéhydes, alcanes, halo-alcanes, alcools, esters, amines, éthers et des composés qui sont métabolisés *in situ* en ces classes de
composés, si le RTF est l'AlkS de *Pseudomonas putida* ou une protéine comprenant des séquences d'acides aminés qui sont identiques à 80% ou plus à l'AlkS.

10. Procédé selon la revendication 9, dans lequel la protéine souhaitée, pour laquelle le niveau d'expression est ajusté, est choisie dans le groupe constitué par la SEAP, une protéine fluorescente, une hormone de croissance humaine, l'interféron alpha, l'interféron bêta, l'interféron gamma, l'insuline, l'érythropoïétine, un activateur du plasminogène tissulaire, une DNAse, un anticorps monoclonal, le Facteur VIII, le Facteur VII, un HAS, l'IL-2, des glucagons, l'EGF, le GCSF, le GMCSF, la thrombopoïétine, la gp160, la HbSAg et une protéine codée par un gène de suppression tumorale.

11. Procédé selon les revendications 9 ou 10, dans lequel le RTF est l'AlcR *d'Aspergillus nidulans* ou une protéine comprenant des séquences d'acides aminés qui sont identiques à 80% ou plus à l'AlcR et le composé destiné à moduler l'expression génique est l'acétaldéhyde.

12. Procédé selon les revendications 9 ou 10, dans lequel le RTF est l'AlkS de *Pseudomonas putida* ou une protéine comprenant des séquences d'acides aminés qui sont identiques à 80% ou plus à l'AlkS et le composé destiné à moduler l'expression génique est choisi dans le groupe constitué par :
l'éthanol ; la méthylamine ; l'éthylamine ; la n-propylamine ; la n-butylamine ; la n-pentylamine ; la n-hexylamine ; la benzylamine ; le n-propanol ; le n-butanol ; le 2-propanol ; le 2-butanol ; le 2-méthylbutyraldéhyde ; l'acétaldéhyde ; le propanal ; l'acétone ; la 2-butanone ; la 2-pentanone ; la 3-pentanone ; la cyclohexanone ; le glycoaldéhyde ; le glyoxal ; le glyoxylate ; l'éthylène glycol ; l'éthanolamine ; l'acétate d'éthyle ; l'éther d'éthyle ; la dicyclopropylcétone ; et des composés qui sont métabolisés *in situ* en ces composés.

13. Procédé selon les revendications 9 ou 10, dans lequel le RTF est la protéine AlcR d'*Aspergillus nidulans* et le composé destiné à moduler l'expression génique est l'acétaldéhyde.

14. Procédé *in vitro* destiné à ajuster le niveau d'expression d'un gène dans une cellule de mammifère, selon la revendication 1, comprenant les étapes consistant à :
a. lier fonctionnellement ledit gène à un promoteur contenant une séquence opérateur, dont la transcription est modulée par le facteur de transcription sensible RTF ;
b. transférer dans ladite cellule de mammifère ledit promoteur contenant une séquence opérateur, lié fonctionnellement audit gène ; et
c. moduler l'expression dudit gène en activant ledit promoteur contenant une séquence opérateur, par l'administration d'un composé auquel est sensible le facteur de transcription sensible RTF spécifique d'une séquence opérateur.

15. Procédé selon la revendication 14, dans lequel le gène code pour une protéine choisie dans le groupe constitué par la SEAP, une protéine fluorescente, une hormone de croissance humaine, l'interféron alpha, l'interféron bêta, l'interféron gamma, l'insuline, l'érythropoïétine, un activateur du plasminogène tissulaire, une DNAse, un anticorps monoclonal, le Facteur VIII, le Facteur VII, un HAS, l'IL-2, des glucagons, l'EGF, le GCSF, le GMCSF, la thrombopoïétine, la gp160 et la HbSAg.

16. Procédé selon la revendication 14, dans lequel le gène est un gène de suppression tumorale.

17. Procédé selon l'une quelconque des revendications 14 à 16, dans lequel le RTF est l'AlcR *d'Aspergillus nidulans* ou une protéine comprenant des séquences d'acides aminés qui sont identiques à 80% ou plus à l'AlcR et le composé auquel est sensible le facteur de transcription sensible RTF spécifique d'une séquence opérateur est l'acétaldéhyde.

18. Procédé selon l'une quelconque des revendications 14 à 16, dans lequel le RTF est l'AlkS de *Pseudomonas putida* ou une protéine comprenant des séquences d'acides aminés qui sont identiques à 80% ou plus à l'AlkS et le composé auquel est sensible le facteur de transcription sensible RTF spécifique d'une séquence opérateur est choisi dans le groupe constitué par : l'éthanol ; la méthylamine ; l'éthylamine ; la n-propylamine ; la n-butylamine ; la n-pentylamine ; la n-hexylamine ; la benzylamine ; le n-propanol ; le n-butanol ; le 2-propanol ; le 2-butanol ; le 2-méthylbutyraldéhyde ; l'acétaldéhyde ; le propanal ; l'acétone ; la 2-butanone ; la 2-pentanone ; la 3-pentanone ; la cyclohexanone ; le glycoaldéhyde ; le glyoxal ; le glyoxylate ; l'éthylène glycol ; l'éthanolamine ; l'acétate d'éthyle ; l'éther d'éthyle ; la dicyclopropylcétone ; et des composés qui sont métabolisés *in situ* en ces composés.

19. Procédé selon la revendication 14, dans lequel le promoteur contenant la séquence opérateur est un site de séquence opérateur spécifique de l'AlcR, le RTF est la protéine AlcR *d'Aspergillus nidulans* et le composé auquel le RTF est sensible est l'acétaldéhyde.

20. Acide nucléique isolé utile pour construire une cellule de mammifère, selon la revendication 1, comprenant un acide nucléique codant pour un RTF, dans lequel le RTF est choisi parmi l'AlcR *d'Aspergillus nidulans,* l'AlkS de *Pseudomonas putida* et des protéines comprenant des séquences d'acides aminés qui sont identiques à 80% ou plus à l'AlcR et l'AlkS, lié fonctionnellement à un promoteur utile pour l'expression du RTF dans ladite cellule de mammifère.

21. Acide nucléique isolé, selon la revendication 20, comprenant une séquence opérateur liée, de manière fonctionnelle, à un promoteur ou à un fragment de celui-ci utile pour une expression génique dépendante du RTF dans ladite cellule de mammifère.

22. Acide nucléique isolé, selon les revendications 20 ou 21, comprenant en outre des éléments génétiques utiles pour construire des vecteurs viraux.


### Sequence Listing

SEQUENCE LISTING

<110> Fussenegger, Martin

Weber, Wilfried

<120> REGULATABLE GENE EXPRESSION IN MAMMALIAN CELLS AND MAMMALS

<130> P314A

<150> EP03405635.8

<151> 2003-09-02

<160> 2

<170> PatentIn version 3.1

<210> 1

<211>31

<212> DNA

<213> Artificial sequence

<220>

<223> Primer for Aspergillus nidulans P alcA promoter

<400> 1

gatcgacgtc ggagctacca tccaataacc c 31

<210> 2

<211> 31

<212> DNA

<213> Artificial Sequence

<220>

<223> Primer for Aspergillus nidulans P AlcA promoter

<400> 2

gatccctgca ggcccgctcg tttgtggctc t 31

### SEQUENCE LISTING

<110> Fussenegger, Martin
   weber, wilfried
<120> REGULATABLE GENE EXPRESSION IN MAMMALIAN CELLS AND MAMMALS
<130> P314A
<150> EP03405635.8
   <151> 2003-09-02
<160> 2
<170> PatentIn version 3.1
<210> 1
   <211> 31
   <212> DNA
   <213> Artificial sequence
<220>
   <223> Primer for Aspergillus nidulans P alcA promoter
<400> 1
   gatcgacgtc ggagctacca tccaataacc c 31
<210> 2
   <211> 31
   <212> DNA
   <213> Artificial Sequence
<220>
   <223> Primer for Aspergillus nidulans P AlcA promoter
<400> 2
   gatccctgca ggcccgctcg tttgtggctc t 31